# EUROPEAN PATENT APPLICATION

(11) **EP 4 613 287 A1**
(43) Date of publication of application: **10.09.2025**
(21) Application number: 23885556.3
(22) Date of filing: 20.10.2023
(51) Int. Cl.: A61K 39/00, A61K 9/14, A61K 39/39, A61K 47/42, A61P 31/00

(54) **ORAL-ADMINISTRATION-TYPE VACCINE COMPOSITION CONTAINING FIBROIN-CONTAINING NANOPARTICLES**

(30) Priority: 31.10.2022 JP 2022174555
(71) Applicant: NATIONAL AGRICULTURE AND FOOD RESEARCH ORGANIZATION, Tsukuba-shi, Ibaraki 305-8517 (JP)
(72) Inventor: SATO, Mitsuru, Tsukuba-shi, Ibaraki 305-8634 (JP); KOJIMA, Katsura, Tsukuba-shi, Ibaraki 305-8634 (JP)
(74) Representative: J A Kemp LLP
(86) International application number: PCT/JP2023/037985
(87) International publication number: WO 2024/095794

(57) **Abstract**

A composition for an oral vaccine comprises nanoparticles containing an antigen and fibroin.

## Description

### [Technical Field]

The present invention relates to an oral-administration-type vaccine composition containing fibroin-containing nanoparticles, and more specifically relates to a composition for an oral vaccine comprising nanoparticles containing an antigen and fibroin or a composition for an oral vaccine comprising nanoparticles containing an antigen, fibroin, and sericin.

### [Background Art]

In recent years, many antibiotics have been used as a countermeasure for preventing or mitigating damage caused by chronic complex infectious diseases that occur in environments for large-scale breeding. The overuse of antibiotics has raised concerns about the emergence of drug-resistant bacteria and food safety issues due to their residue in food. The development of safe and useful animal vaccines as alternatives to these antibiotics has been in progress. Furthermore, in the development of such vaccines, oral vaccines that can reduce the burden on animals and the risk to workers are more desirable than injection vaccines.

In addition, it is said that vaccination by injection can induce systemic protective immunity and sensitization, but cannot provide protection through the respiratory and digestive mucosal tissues, which may act as the entry points for pathogens, and is less effective in preventing infection, especially in the cases of infectious diseases.

Moreover, it is very difficult to use an injection vaccine as an oral vaccine. This is because when orally administered, a vaccine must pass through the stomach, where the vaccine is broken down by the highly acidic environment and digestive enzymes such as pepsin, and therefore often fails to accurately and effectively induce an antigen-specific immune response.

### [Citation List]

### [Patent Literature]

[PTL 1] Japanese Unexamined Patent Application Publication No. 2005-97229

### [Non Patent Literature]

[NPL 1] Andreas S Lammel, et al., Biomaterials, June 2010, Vol. 31, No. 16, pp. 4583 to 4591
[NPL 2] Zheng Zhao, et al., Int J Mol Sci., March 4, 2015, Vol. 16, No. 3, pp. 4880 to 4903
[NPL 3] Fatemeh Rezaei, et al., Biomater Sci., April 7, 2021, Vol. 9, No. 7, pp. 2679 to 2695

### [Summary of Invention]

### [Technical Problem]

The present invention has been made in consideration of the problems in the related art, and has an object to provide a composition for a vaccine that can induce antibody production specific to an antigen in animals through oral administration.

### [Solution to Problem]

In order to achieve the above object, the present inventors came up with an idea of using silk protein as a carrier for an oral vaccine.

Silk can be processed into powder, film, sponge, and so on by removing the outer sericin layer through a degumming process and dissolving the resulting fibroin fibers into an aqueous solution. Moreover, it is also possible to produce fibroin nanoparticles from a fibroin aqueous solution by salting out, desalination, or the like. There have been made attempts to use such nanoparticles as a carrier for a delivery system of a drug or the like (NPTs 1 to 3).

It was also reported that when cocoons produced by genetically recombinant silkworms expressing an antigenic protein were physically crushed without degumming and the resulting silk powder was injected subcutaneously into mice together with complete Freund's adjuvant, the antibody titers in the blood consequently increased (PTL 1).

However, as a result of preparing silk powder according to the method described in PTL 1 and orally administering the silk powder to mice, the present inventors found that antibody production against the silk components was predominantly induced, suggesting that it is difficult to efficiently induce antibody production against a target antigen.

Therefore, the present inventors conducted further intensive research and prepared silk nanoparticles containing an antigenic protein by salting out from a mixture solution of the antigenic protein with a silk solution in which degummed fiber was dissolved (a solution containing fibroin) or a silk solution in which non-degummed cocoon fiber was dissolved (a solution containing sericin and fibroin). Then, the present inventors found that oral administration of these nanoparticles could induce antibody production specific to the antigenic protein. In particular, in the antibody production, the induction of antigen-specific production of both IgA, which exhibits activation of mucosal immunity, and IgG, which exhibits activation of systemic immunity, was observed. In other words, the present inventors clarified that the nanoparticles are an excellent oral vaccine carrier that can deliver an antigen to the intestinal tracts (small intestine and large intestine) while avoiding decomposition of the antigen by gastric acid and digestive enzymes in the stomach, thereby making it possible to activate mucosal immunity and also induce systemic immunity, and thus completed the present invention.

Specifically, the present invention provides the following aspects.
[1] A composition for an oral vaccine comprising nanoparticles containing an antigen and fibroin.
[2] The composition for an oral vaccine according to [1], wherein the nanoparticles further contain sericin.
[3] The composition for an oral vaccine according to [1], wherein the nanoparticles are precipitated by salting out from a mixture solution of a dissolving solution of degummed silk with the antigen.
[4] The composition for an oral vaccine according to [2], wherein the nanoparticles are precipitated by salting out from a mixture solution of a dissolving solution of non-degummed silk fiber with the antigen.

### [Advantageous Effects of Invention]

According to the present invention, it is possible to induce antibody production specific to an antigen in animals through oral administration.

### [Brief Description of Drawings]

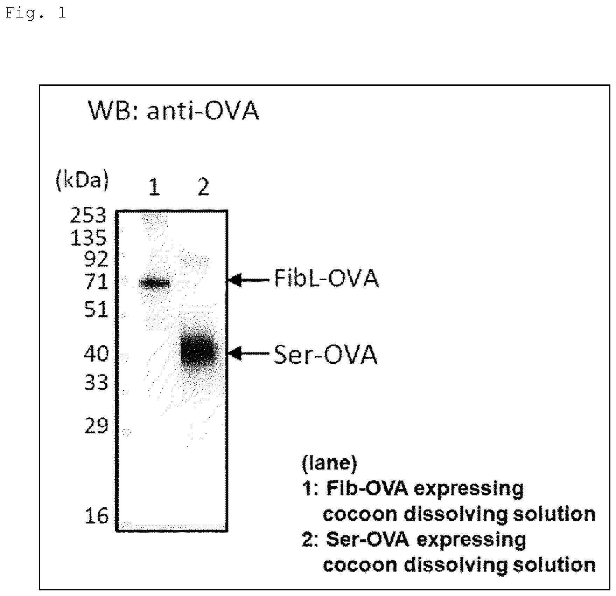

[Fig. 1] Fig. 1 is a photograph showing results of Western blotting using anti-ovalbumin (OVA) antibody to detect expression of a fusion protein of fibroin L chain (FibL) with OVA (FibL-OVA) or expression of free OVA in a sericin layer (Ser-OVA) in cocoons produced by genetically recombinant silkworms.

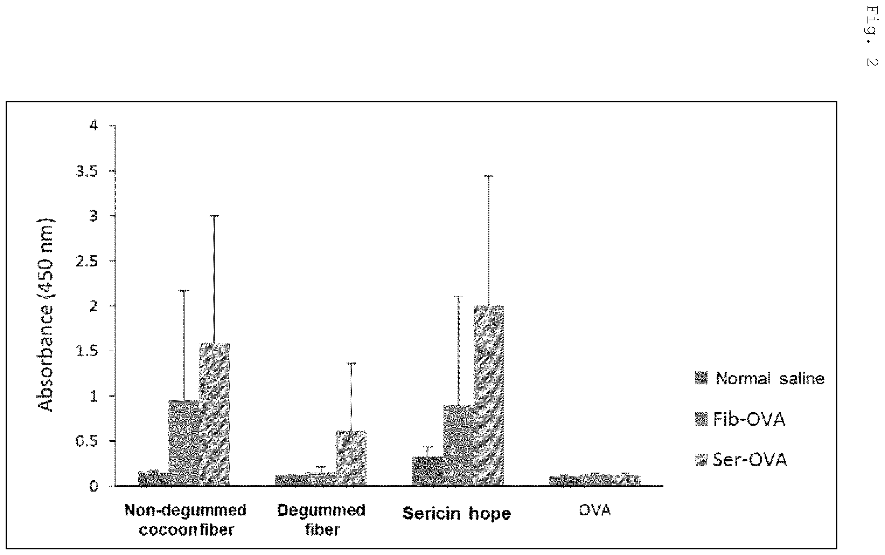

[Fig. 2] FIG. 2 is a graph showing results of evaluating antibody titers in the serum of mice subjected to oral administration with a suspension of fragments of FibL-OVA expressing cocoons or Ser-OVA expressing cocoons in normal saline. In Fig. 2, "NON-DEGUMMED COCOON FIBER" , "DEGUMMED FIBER", "SERICIN HOPE", and "OVA" on the horizontal axis show results of ELISA using an assay plate coated with an aqueous solution of non-degummed cocoon fibers, a dissolving solution of degummed cocoon fibers, an aqueous solution of sericin hope cocoons (containing only sericin, not fibroin), and an OVA solution, respectively. In Fig. 2, "NS" indicates results of a test group to which only normal saline was orally administered as a control.

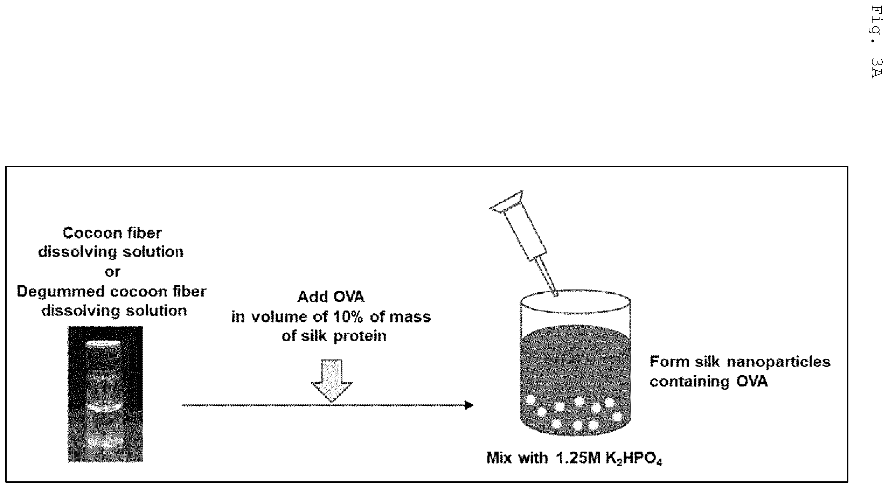

[Fig. 3A] Fig. 3A is a diagram showing steps of preparing silk nanoparticles by mixing a non-degummed cocoon fiber dissolving solution or a degummed cocoon fiber dissolving solution with antigenic protein (OVA).

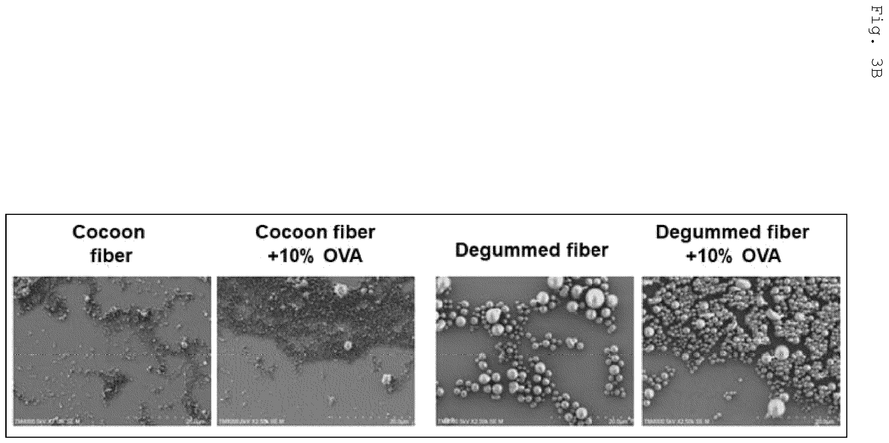

[Fig. 3B] Fig. 3 includes photographs showing observation results of the OVA-containing silk nanoparticles with a scanning electron microscope (SEM). In Fig. 3B, "COCOON FIBER" shows an observation result of silk nanoparticles prepared from a non-degummed cocoon fiber dissolving solution alone, "COCOON FIBER+10% OVA" shows an observation result of silk nanoparticles prepared by mixing OVA with the dissolving solution, "DEGUMMED FIBER" shows an observation result of silk nanoparticles prepared from a degummed fiber dissolving solution alone, and "DEGUMMED FIBER+10% OVA" shows an observation result of silk nanoparticles prepared by mixing OVA with the dissolving solution.

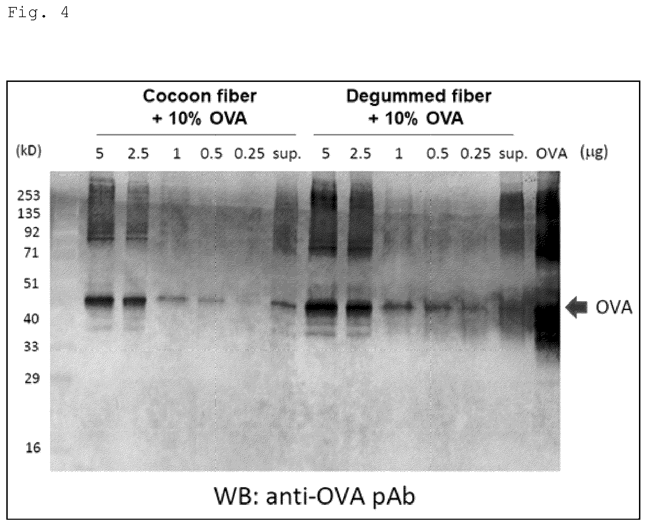

[Fig. 4] Fig. 4 is a photograph showing results of detecting the OVA incorporated into the silk nanoparticles by Western blotting using anti-OVA antibody.

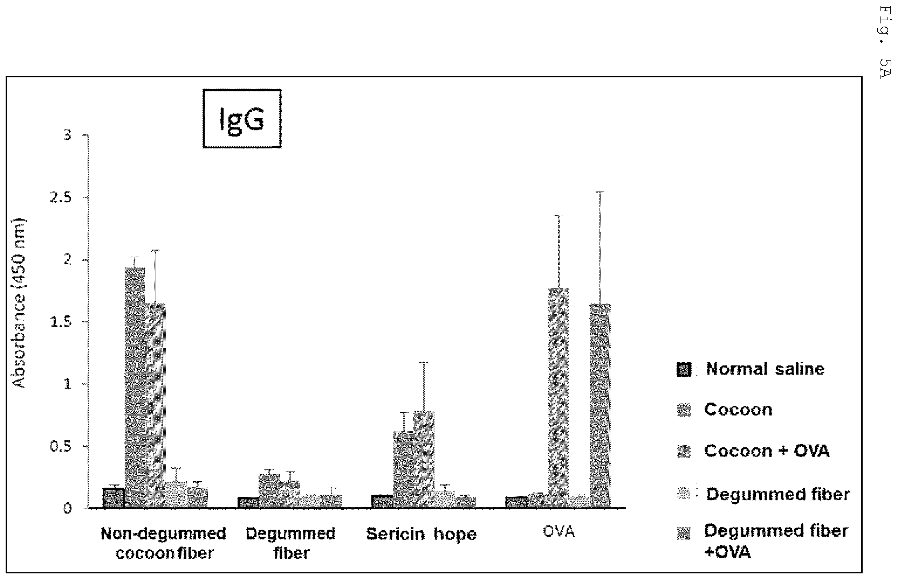

[Fig. 5A] Fig. 5A is a graph showing results of ELISA analyses of an IgG amount in the serum of mice subjected to oral administration with the OVA-containing silk nanoparticles. In Fig. 5A, "NON-DEGUMMED COCOON FIBER" , "DEGUMMED FIBER", "SERICIN HOPE", and "OVA" on the horizontal axis show the results of ELISA using an assay plate coated with an aqueous solution of non-degummed cocoon fibers, a dissolving solution of degummed cocoon fibers, an aqueous solution of sericin hope cocoons (containing only sericin, not fibroin), and an OVA solution, respectively. In the legends, "NS" indicates results of a test group to which normal saline was orally administered, "COCOON" indicates results of a test group to which silk nanoparticles prepared from a non-degummed cocoon fiber dissolving solution alone were orally administered, "COCOON+OVA" indicates results of a test group to which silk nanoparticles prepared by mixing OVA with the non-degummed cocoon fiber dissolving solution were orally administered, "DEGUMMED FIBER" indicates results of a test group to which silk nanoparticles prepared from a degummed cocoon fiber dissolving solution alone were orally administered, and "DEGUMMED FIBER+OVA" indicates results of a test group to which silk nanoparticles prepared by mixing OVA with the degummed cocoon fiber dissolving solution.

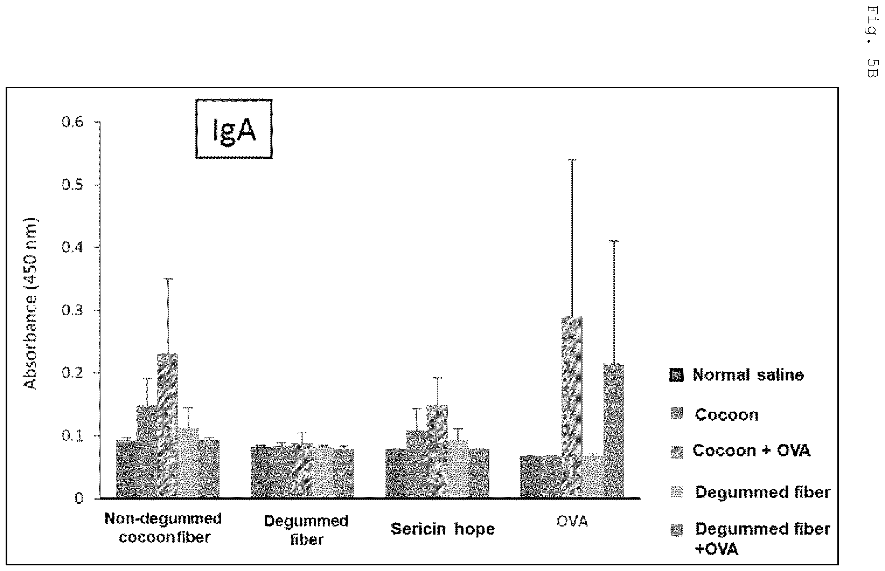

[Fig. 5B] Fig. 5B is a graph showing results of ELISA analyses of an IgA amount in the serum of mice to which the OVA-containing silk nanoparticles were orally administered. The legends in Fig. 5B are the same as those in Fig. 5A.

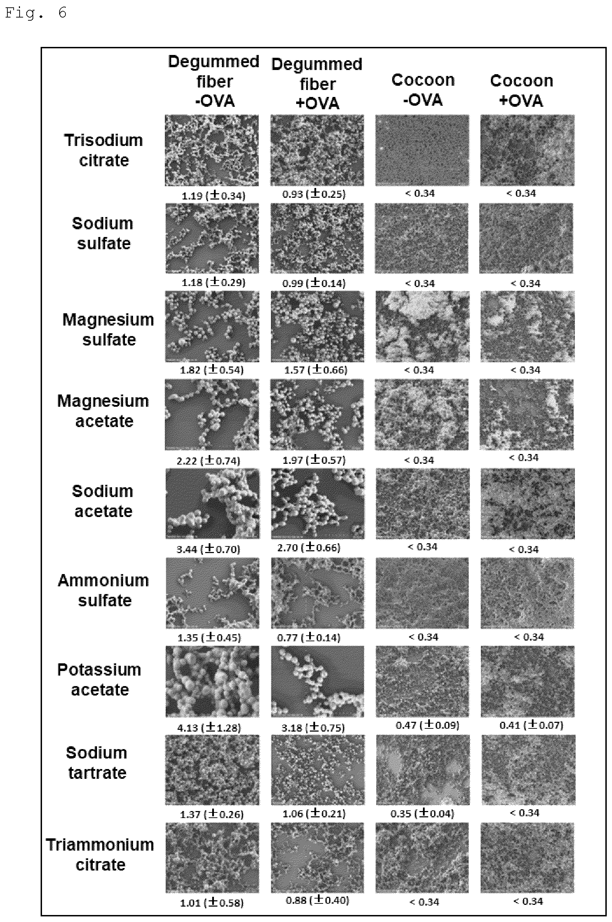

[Fig. 6] Fig. 6 includes photographs showing results of SEM observations of silk nanoparticles prepared by salting out using aqueous solutions in which various inorganic and organic salts were dissolved. In Fig. 6, "DEGUMMED FIBER" indicates nanoparticles prepared from degummed cocoon fiber, "COCOON" indicates nanoparticles prepared from non-degummed cocoon fiber, and "-OVA" or "+OVA" indicates nanoparticles prepared with or without addition of antigenic protein (OVA). The left side specifies the types of inorganic salts and organic salts used in the salting out. Below each photograph, an average particle size (diameter, µm) is presented and a numeric value in parentheses indicates a standard deviation (SD). The average particle size herein is an average value calculated from the maximum diameters of respective particles (the diameter of a smallest circle enclosing each particle) present in one field of view of the SEM observation. The number of particles detected was 50 or more except for the following two types of patterns. (Pattern 1) 5 to 20 particles were detected in the case where only particles smaller than the measurement lower limit (examples with a particle size < 0.34 µm in Fig. 6) were present in one field of view of the SEM observation. (Pattern 2) All particles were detected as far as possible in the case where 50 or fewer measurable particles were present in one field of view (sodium acetate: DEGUMMED FIBER-OVA, potassium acetate: DEGUMMED FIBER+OVA, and so on).

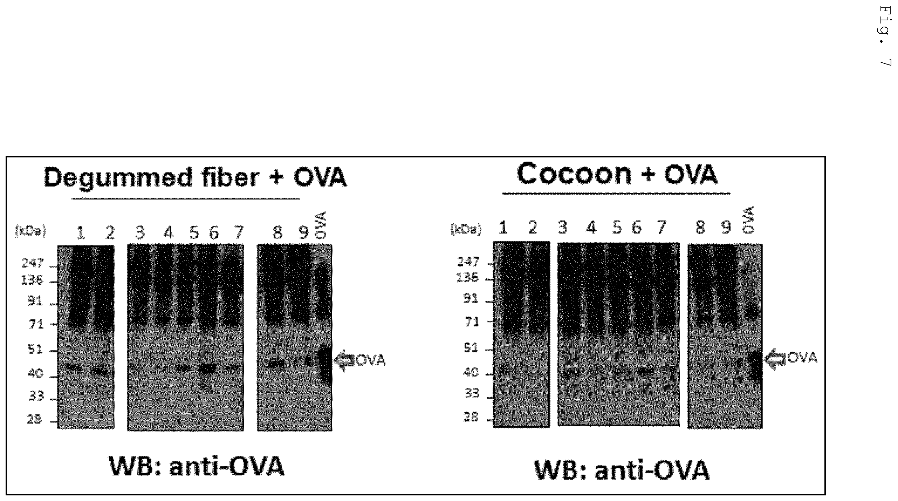

[Fig. 7] Fig. 7 includes photographs showing results of detecting the OVA incorporated into silk nanoparticles prepared by using the aqueous solution in which each of the various inorganic salts and organic salts was dissolved by Western blotting using anti-OVA antibody. In Fig. 7, "DEGUMMED FIBER+OVA" indicates silk nanoparticles prepared from a degummed cocoon fiber dissolving solution with addition of antigenic protein (OVA), and "COCOON+OVA" indicates silk nanoparticles prepared from non-degummed cocoon fiber with addition of the OVA. In each lane, an inorganic salt or organic salt used in the salting out is as follows. The lane with "OVA" is a lane as a positive control. Lane No.1: trisodium citrate, Lane No.2: sodium sulfate, Lane No.3: magnesium sulfate, Lane No.4: magnesium acetate, Lane No.5: sodium acetate, Lane No.6: ammonium sulfate, Lane No.7: potassium acetate, Lane No.8: sodium tartrate, and Lane No.9: triammonium citrate.

### [Description of Embodiments]

As shown in Examples to be described below, the present inventors clarified that oral administration of antigen-containing nanoparticles prepared from a mixture solution of a dissolving solution of silk (a solution containing sericin and fibroin) with an antigen can induce the antibody production specific to the antigen.

The present inventors further clarified that oral administration of antigen-containing nanoparticles prepared from a mixture solution of a dissolving solution of degummed silk (a solution containing fibroin) with an antigen can induce the antibody production specific to the antigen.

Therefore, the present invention relates to a composition for an oral vaccine comprising nanoparticles containing an antigen and fibroin, and the present invention also relates to a composition for an oral vaccine comprising the nanoparticles further containing sericin.

### <Nanoparticles according to Present Invention>

In the present invention, "fibroin" contained in nanoparticles means at least one type of protein selected from the group consisting of fibroin L chain, fibroin H chain, and fibrohexamerin (P25), or a partial peptide of the protein. In addition, the "fibroin" according to the present invention also includes complexes of the above proteins. An example of the complexes is a naturally existing complex (for example, a heterodimer in which fibroin L chain and fibroin H chain are disulfide-bonded or a complex in which six molecules of such dimers and one molecule of fibrohexamerin are non-covalently bonded).

In the present invention, "sericin" means at least one type of protein selected from the group consisting of sericin 1, sericin 2, sericin 3, and sericin 4 or a partial peptide of the protein. In addition, the "sericin" according to the present invention also includes complexes of the above proteins. An Example of the complexes is a naturally existing complex (for example, a sericin layer containing sericin 1, sericin 2, and sericin 3, or a fragment of the layer.

In the nanoparticles according to the present invention, the fibroin and the sericin may be contained in the form of a complex. Moreover, the fibroin and the sericin may be natural proteins derived from silk or recombinant proteins. Examples of the "natural proteins" include proteins produced by Lepidoptera (domestic silkworms (genus *Bombyx,* species *mori*)*,* wild silkworms (genus *Antheraea,* species *pernyi, yamamai, militta, assama,* and the like, and genus *Philosamia,* species *cynthia ricini,* bagworm moths, and the like), spiders (order Araneae), and arthropods belonging to the orders Hymenoptera or Diptera. The "recombinant protein" means protein prepared by a genetic engineering technique, and can be prepared, for example, by expressing a polynucleotide encoding the above-mentioned protein in a host cell such as E. coli, yeast, insect cell, or animal cell, or in a cell-free expression system such as an E. coli extract, a rabbit reticulocyte extract, or a wheat germ extract.

In the present invention, "silk" means fiber composed of fibroin fiber made of the above-mentioned fibroin and a sericin layer that envelops the fiber. In the case of a naturally-derived silk, the silk means fiber prepared from a cocoon or the like produced by any of the above-mentioned arthropods, and examples of the silk according to the present invention include cocoon shell, cocoon fiber, raw silk, and silk fiber. The "degumming" means a process of removing sericin from silk and is not particularly limited as long as the process can remove sericin. Examples of the process include alkali degumming, acid degumming, soap degumming, enzyme degumming, or a combination of these (for example, soap-alkali degumming). Among these, the alkali degumming is preferred as a method which is preferable from the viewpoint of cost and which has been industrially proven as effective. The alkali degumming means a process of boiling silk in the presence of an alkali. The alkali used in this process is usually a weak alkali, such, for example, as sodium carbonate, sodium bicarbonate, or sodium silicate. The alkali degumming using the sodium carbonate among these is most preferred from the viewpoints of cost and reagent solubility.

In the present invention, in order to prepare nanoparticles, first, fibroin and additionally sericin as well as degummed silk or non-degummed silk are dissolved.

A solvent to be used in such dissolution is not particularly limited, and water and/or an organic solvent or the like may be used. As the organic solvent, for example, lower alcohols (such as ethanol, methanol, and isopropanol) and glycols (such as propylene glycol and diethylene glycol) may be used. Aprotic solvents such as dimethyl sulfoxide, dimethylformamide, dimethylacetamide, N-methylmorpholine-N-oxide may also be used. However, from the viewpoints of safety and environmental impact, a mixture solvent of water and a lower alcohol is preferred, and a mixture solvent of water and ethanol is more preferred.

It is preferable to add an inorganic salt to the solvent. The inorganic salt is not particularly limited, and for example, salts of alkali metals and alkaline earth metals may be used. Specific inorganic salts include lithium bromide, potassium bromide, calcium bromide, lithium chloride, potassium chloride, calcium chloride, lithium thiocyanate, potassium thiocyanate, and so on. From the viewpoint of an ability to easily and efficiently dissolve fibroin, sericin, and an antigenic protein while suppressing their decomposition, lithium bromide, lithium thiocyanate, and calcium chloride are preferred, and lithium bromide is more preferred.

In the present invention, the concentration (final concentration) of the inorganic salt added to the solvent is not particularly limited, but may be appropriately set to, for example, 3M or more, 4M or more, 5M or more, 6M or more, 7M or more, 8M or more, 9M or more, or 10M or more. The pH of the solvent is not particularly limited but is preferably pH 7 or more (for example, pH 8 or pH 9).

The temperature for the dissolution is not particularly limited but is preferably 100°C or less, and more preferably 55°C or less (such as 37°C or room temperature (for example, 25°C)). The dissolving solution thus prepared may be subjected to, for example, a dialysis process for removing the inorganic salt (desalting). The dialysis solution used for the process is not particularly limited, but may be deionized water, RO water, a solution containing such water and a buffer (such as 1 mM Tris-HCl (pH 9) or PBS), or any of the above-mentioned solvents.

In the present invention, the dissolving solution thus prepared is mixed with an antigen to be described later, and particles are formed from the resulting mixture. The particle formation method is not particularly limited as long as the method can prepare the above nano-sized particles. Examples of the method include salting out (salting-out method, see Lammel, A.S. et al., Biomaterials 2010, 31, 4583-4591 and others), a desolvation method such as ethanol precipitation (coacervation method, see Kundu, J. et al., Int. J. Pharm., 2010, 388, 242-250, Zhang, Y.Q. et al., J. Nanopart. Res., 2007, 9, 885-900, Cao, Z. et al., Soft Matter, 2007, 3, 910-915, Shi, P.J. et al., Int. J. Pharm., 2011, 410, 282-289, and others), supercritical fluid technology (see Zhao, Z. et al., Ind. Eng. Chem. Res., 2013, 52, 3752-3761, and others), an electrospray method (See Gholami, A. et al., J. Nanopart. Res., 2011, 13, 2089-2098, Qu, J. et al., Mater. Sci. Eng. C: Mater. Biol. Appl., 2014, 44, 166-174, and others), mechanical crushing (See Rajkhowa, R. et al., Powder Technol., 2008, 185, 87-95, Rajkhowa, R. et al., Powder Technol., 2009, 191, 155-163, Kazemimostaghim, M. et al., Powder Technol., 2013, 241, 230-235, Kazemimostaghim, M. et al., Powder Technol., 2013, 249, 253-257, and others), a microemulsion method (See Myung, S. J. et al., Macromol. Res., 2008, 16, 604-608, and others), an electrical control method (Huang, Y.L. et al., Chin. Sci. Bull., 2011, 56, 1013-1018, and others), capillary microdot technology (see Gupta, V. et al., Int. J. Nanomed., 2009, 4, 115-122, and others), and a PVA mixed film method (see Wang, X. et al., Biomaterials 2010, 31, 1025-1035, and others).

Among these particle formation methods, from the viewpoints of the size, cost and yield of the obtained nanoparticles, the ease of reducing the denaturation and decomposition of an antigenic protein, and the ease of avoiding the incorporation of other components into the nanoparticles, preferred are the salting out and the desolvation method, with the salting out being more preferred. In the salting out, a salt for use is not particularly limited as long as it can be added to the above-mentioned dissolving solution to precipitate the antigen, the fibroin, and so on, and either an inorganic salt or an organic salt may be used. Examples of the usable inorganic salt include salts of alkali metals or alkaline earth metals, and ammonium salts, and specific examples thereof include dipotassium phosphate, monopotassium phosphate, monosodium phosphate, disodium phosphate, magnesium phosphate, magnesium sulfate, sodium chloride, potassium chloride, ferrous sulfate, manganese sulfate, copper sulfate, calcium carbonate, sodium sulfate, ammonium sulfate, and the like. Examples of the organic salt include trisodium citrate, triammonium citrate, magnesium acetate, sodium acetate, potassium acetate, and sodium tartrate. Among these salts, dipotassium phosphate, disodium phosphate, monopotassium phosphate, and monosodium phosphate are preferred, and dipotassium phosphate is more preferred, from the viewpoints that they are unlikely to become harmful to inoculated animals, and that they allow easy and efficient recovery of nanoparticles without needing dilution.

In the present invention, the concentration (final concentration) of the salt added to the mixture solution of the antigen with the above-mentioned dissolving solution is not particularly limited as long as the antigen, the fibroin, and so on can be precipitated by the salting out, but may be, for example, 0.8 M or more, preferably 1 M or more, and more preferably 1.2 M or more. Such salting out can be carried out by adding a saturated aqueous solution of a salt to the antigen and the above-mentioned dissolving solution and mixing them. The temperature for the salting out is not particularly limited but is usually room temperature or less (for example, 25°C or 4°C).

In the present invention, for example, the precipitate thus obtained is washed with water and crushed to prepare nanoparticles. Such preparation method is not particularly limited, and examples thereof include ultrasonic treatment and stirring.

The "nanoparticles" according to the present invention are microparticles that contain such silk protein as a carrier, and may be in any form. The particle size thereof is nano-sized or micro-sized. The term "nano-sized or micro-sized" means that the average particle size is less than 5 µm, preferably less than 4 µm, more preferably less than 3 µm, even more preferably less than 2 µm, much more preferably 1 µm, further preferably 900 nm or less, more preferably 800 nm or less, even more preferably 700 nm or less, much more preferably 600 nm or less, or even further preferably 500 nm or less (for example, 400 nm or less, 350 nm or less, 300 nm or less, 250 nm or less, 200 nm or less, 150 nm or less, or 100 nm or less). The "average particle size" means, for example, the average value of particle sizes measured by observation with a scanning electron microscope (SEM), and more specifically means the average value calculated from the maximum diameters of the respective particles (the diameter of a smallest circle enclosing each particle) present in one field of view of the SEM observation. The number of particles to be detected is, for example, 5 to 100 (for example, 5 to 10, 5 to 20, 5 to 30, 5 to 40, 5 to 50, or 50 or more). Instead, all the particles present in one field of view may be detected.

Regarding the content of each type of protein in the nanoparticles according to the present invention, in the case of nanoparticles containing an antigen and fibroin (for example, nanoparticles prepared from degummed silk), the content of the antigen in the nanoparticles is preferably 0.05 to 30% by mass, more preferably 0.1 to 20% by mass, and even more preferably 0.1 to 10% by mass, relative to the content of the fibroin in the nanoparticles. The mass ratio of the fibroin to the sericin in the silk is about 3:1 (7:3 to 8:2). Moreover, in the degumming, the sericin may not be completely removed from the silk depending on the method, conditions, and the like. For this reason, in this case, the nanoparticles according to the present invention may contain, as an impurity, 0.1 to 20% by mass of sericin relative to the content of the fibroin in the nanoparticles.

In the case of nanoparticles according to the present invention containing an antigen, fibroin, and sericin (for example, nanoparticles prepared from non-degummed silk), the content of the antigen in the nanoparticles is preferably 0.05 to 30% by mass, more preferably 0.1 to 20% by mass, and even more preferably 0.1 to 10% by mass, relative to the content of the fibroin and the sericin in the nanoparticles. The mass ratio between the fibroin and the sericin (the mass of the fibroin in the nanoparticles:the mass of the sericin in the nanoparticles) is preferably 60 to 90:10 to 40, and more preferably 65 to 85:15 to 35.

The "antigen" contained in the nanoparticles of the present invention is not particularly limited as long as the antigen can induce an immune response in an animal, and examples thereof include viruses, bacteria, parasites, fungi, rickettsia, chlamydia, prions, cancer cells, or molecules derived from these (for example, proteins, nucleic acids, sugars, and lipids).

The viruses, bacteria, parasites, fungi, rickettsia, chlamydia, and so on may be either attenuated (live vaccines) or inactivated (inactivated vaccines) for use as the antigen. In addition, toxoids, capsids, surface protein, or the like derived from these may also be used as the antigen.

The virus is not particularly limited, as long as the virus infects animals and causes a disease, and examples thereof include adenovirus, influenza virus, Ebola virus, Nipah virus, papillomavirus, human immunodeficiency virus, hepatitis virus (types A, B, C, D, E, F, G, and so on), measles virus, rubella virus, poliovirus, rotavirus, norovirus, sapovirus, enterovirus, rabies virus, yellow fever virus, varicella-zoster virus, mumps virus, cytomegalovirus, coronavirus, polyomavirus, herpes virus, Japanese encephalitis virus, dengue virus, Marburg virus, parvovirus, Lassa virus, hantavirus, *Thogotovirus,* Dhori virus, Newcastle disease virus, *Togaviridae,* paramyxovirus, orthomyxovirus, poxvirus, *Reoviridae,* and foot-and-mouth disease virus.

The bacterium is not particularly limited as long as the bacterium infects animals and causes a disease, and examples include *Clostridium tetani, Streptococcus pyogenes, Staphylococcus aureus, Enterococci, Listeria monocytogenes,* pathogenic *Escherichia coli, Bordetella pertussis, Corynebacterium diphtheriae, Klebsiella pneumoniae, Proteus* bacteria, *Neisseria meningitidis, Pseudomonas aeruginosa, Serratia marcescens, Neisseria gonorrhoeae, Enterobacter* bacteria, *Citrobacter* bacteria, *Mycoplasma, Clostridium, Mycobacterium tuberculosis, Vibrio cholerae, Yersinia pestis, Shigella, Bacillus anthrax, Treponema pallidum, Legionella* bacteria, *Leptospira* bacteria, *Helicobacter pylori, Borrelia* bacteria, and *Haemophilus influenzae.* The parasite is not particularly limited as long as it parasitizes animals and causes a disease, and examples thereof include malaria parasites, *Toxoplasma gondii, Leishmania, Trypanosoma, Cryptosporidium, Echinococcus, Schistosoma, Filaria,* and roundworms.

The fungus is not particularly limited as long as it infects animals and causes a disease, and examples thereof include *Candida* fungi, *Aspergillus* fungi, *Cryptococcus* fungi, *Histoplasma* fungi, tinea fungi, *Pneumocystis* fungi, and *Coccidioides* fungi.

### <Vaccine Composition and Other of Present Invention>

The composition of the present invention is a composition for an oral vaccine containing the silk particles described above. Such composition may contain pharmacologically acceptable additives in addition to the silk particles. In the case where the composition of the present invention is in the form of a solid preparation, for example, the "pharmacologically acceptable additives" include excipients, binders, lubricants, film coating agents, and so on. In the case where the composition of the present invention is in the form of a liquid preparation, the additives include tonicity adjusting agents, solubilizers, preservatives, stabilizers, suspending agents, emulsifiers, and so on. The composition of the present invention may further contain an adjuvant. Examples of the adjuvant include inorganic substances such as incomplete Freund's adjuvant, complete Freund's adjuvant, and aluminum gel adjuvant, microorganisms or substances derived from microorganisms (such as BCG, muramyl dipeptide, *Bordetella pertussis,* pertussis toxin, and cholera toxin), surfactants (such as saponin and deoxycholic acid), emulsions of oily substances (such as mineral oil, vegetable oil, and animal oil), and alum.

The present invention also provides a method for inducing antibody production specific to the above antigen in an animal by orally administering the nanoparticles according to the present invention or the composition for an oral vaccine of the present invention to the animal, as will be described below in Examples. In other words, this method is a method for preventing a disease related to the antigen (such as an infectious disease) or reducing the symptom thereof.

In the present invention, an "animal" to be subjected to the oral administration is not particularly limited, and may be either a human or a non-human animal. The "non-human animal" is not particularly limited, but may be any of various types of livestock, pets, experimental animals, and so on. Specific examples thereof include, but are not limited to, pigs, cows, horses, sheep, goats, chickens, wild ducks, ostriches, domestic ducks, dogs, cats, rabbits, hamsters, mice, rats, monkeys, and so on.

Furthermore, the dosage of the nanoparticles according to the present invention or the composition for an oral vaccine of the present invention may be determined as appropriate by a person skilled in the art depending on a type of antigen, or a type, age, weight, health condition, or the like of an administration target. In addition, the number of administrations may be a single administration or multiple administrations, and this number may also be determined as appropriate by a person skilled in the art depending on the type of antigen and so on.

### [Examples]

Hereinafter, the present invention will be described more specifically on the basis of Examples and Comparative Examples; however, the present invention is not limited to Examples below. Examples were conducted by using materials and methods described below.

### (Materials and Methods for Experiment)

### 1. Oral Administration Test in Mice Using Cocoons Produced by Recombinant Silkworms as Immunogen

According to the method described in PTL 1 (JP 2005-97229 A), cocoon fibers of genetically recombinant silkworms expressing an antigenic protein were crushed, and whether oral administration of the obtained fragments induced antibody production specific to the antigen was evaluated. Specifically, the test was carried out as described below.

1-1. Process of Crushing Cocoons Produced by Recombinant Silkworms expressing fibroin L chain-fused OVA protein (FibL-OVA) or Cocoons Produced by Recombinant Silkworms expressing free OVA in the sericin layer (Ser-OVA).

Silk fragments with particle sizes of several tens to several hundreds of µm were prepared by cutting the cocoons expressing FibL-OVA and Ser-OVA into 0.5 cm squares, followed by freeze-crushing under liquid nitrogen conditions using Multi-Beads Shocker MB3200 (S) (Yasui Kikai Co., Ltd.) (crushing for 3 seconds followed by freezing in liquid nitrogen for 3 minutes was repeated in 15 cycles), and then wet cooling crushing (crushing for 30 seconds followed by cooling (0°C) for 60 seconds was repeated in 40 cycles) with addition of water (solvent).

### 1-2. Oral Administration Test of FibL-OVA Expressing Silk Fragments or Ser-OVA Expressing Silk Fragments to Mice

The silk fragments were suspended in normal saline to a concentration of 50 mg/ml calculated from the mass of the cocoons, and the obtained sample was orally administered using an oral probe at a dosage of 10 ml/kg (mouse body weight) per administration. The equal volume of normal saline was orally administered as a control. After three consecutive days of oral administrations, the sample was orally administered three times at weekly intervals from the first administration, and whole blood was collected under isoflurane inhalation anesthesia on the next day after the final administration. Three 6-week-old female ICR mice were used in each of the test and control groups.

### 1-3. ELISA Using Serum of Mice Subjected to Oral Administration with FibL-OVA Expressing Silk Fragments or Ser-OVA Expressing Silk Fragments

In a 96-well assay plate, 100 µl of a dissolving solution of non-degummed cocoons or a dissolving solution of degummed fibers from non-recombinant silkworms w1-pnd, a dissolving solution of sericin hope (consisting of sericin only) (0.25 mg/ml in each dissolving solution), or an OVA solution (0.625 mg/ml) was dispensed into each well, which cell was then coated with it at 4°C overnight. Each well was blocked with an assay diluent (Biolegend) (left to stand at room temperature for 60 minutes). After washing with PBS-T (PBS containing 0.05% Tween 20), the resultant was reacted with the mouse serum diluted at 1/200 with the assay diluent (room temperature, 90 minutes). After washing, the resultant was reacted with HRP-labeled anti-mouse immunoglobulin antibody (Dako) (room temperature, 60 minutes). After washing, an ELISA POD substrate TMB solution (NACALAI TESQUE, INC.) was added. After color development, the reaction was stopped by adding 2N H₂SO₄, and the absorbance (450 nm) was measured (Bio-Rad, iMark Microplate Reader).

### 2. Preparation of Antigenic Protein-Containing Silk Nanoparticles by Salting-out Method

### 2-1. Preparation of Cocoons and Degummed Fibers

As the cocoons, W1-pnd cocoons were used. As the degummed fibers, used were raw silk fibers named Gunma 200 degummed with carbon dioxide (treated with 0.02 M sodium carbonate solution at 98°C for 30 minutes).

### 2-2. Preparation of Cocoon and Degummed Fiber Aqueous Solutions

The cocoons or the degummed fibers were washed with 70% ethanol and then with 0.1% SDS aqueous solution, and thereafter were rinsed thoroughly with RO water. For 2 g of the degummed fibers, an aqueous solution was prepared by adding 2 ml of 70% ethanol, 18 ml of 10 M LiBr, 100 mM Tris-HCl (pH 9.0) and mixing them vigorously at 37°C. For 1 g of the cocoons, an aqueous solution was prepared by adding 4 ml of 70% ethanol and 36 ml of 10 M LiBr, 100 mM Tris-HCl (pH 9.0) and mixing them vigorously at 37°C. Each of the obtained silk aqueous solutions was enclosed in a dialysis membrane (EIDIA Co., Ltd., UC24-32-100) and dialyzed against RO water. The dialysis was continued while the RO water was exchanged, until the electrical conductivity of the external dialysis fluid at or after 4 hours of the dialysis became 0.3 µS or less. The silk solution after the dialysis was collected in a centrifuge tube and centrifuged (TOMY MX-307) at 12,000 rpm, 25°C, for 10 minutes to remove solid matters, and the resultant was then diluted with RO water to prepare a 10 mg/ml aqueous solution.

### 2-3. Preparation of Silk Nanoparticles (1)

To 50 ml of the 10 mg/ml silk aqueous solution (the cocoons or the degummed fibers), 50 ml of 1 mg/ml (w/w) OVA (FUJIFILM Wako Pure Chemical Corporation, 012-09885) or RO water was added and mixed, and then was left to stand at room temperature for 20 minutes to prepare an undiluted solution for nanoparticles.

The prepared four types of undiluted solutions for nanoparticles have the following compositions:
(1) Cocoon Dissolving Solution (5 mg/ml);
(2) Cocoon+OVA Dissolving Solution (5 mg/ml Cocoon Dissolving Solution + 0.5 mg/ml OVA Solution);
(3) Degummed Fiber Dissolving Solution (5 mg/ml); and
(4) Degummed Fiber+OVA Dissolving Solution (5 mg/ml Degummed Fiber Dissolving Solution + 0.5 mg/ml OVA Solution).

In a 500 ml centrifuge bottle, 360 ml of 1.25 M K₂HPO₄ was placed, to which 90 ml of each of the above undiluted solutions for nanoparticles was added, and the resultant was left to stand for 30 minutes. After that, the resultant was mixed by slow inversion and left to stand at 4°C over two nights to form nanoparticles. After being left to stand, the resultant was centrifuged (Himac RC-20, 4,000 rpm, 30 minutes, 25°C) to remove the supernatant, and an operation of washing the precipitate by centrifugation with addition of 500 ml of RO water was repeated twice. An operation of suspending the resulting precipitate in a small volume of RO water, collecting the suspension in a 50 ml tube, diluting the collected suspension up to 50 ml with RO water, removing the supernatant by centrifugation (TOMY MX-307; 5,000 rpm, room temperature, 10 minutes), and again washing the resultant with RO water was repeated twice. Then, 10 ml of RO water was added to suspend the precipitate, followed by ultrasonic disruption (BRANSON SONIFIER) to obtain a silk nanoparticle solution. In addition, a portion of the silk nanoparticle solution was taken and dissolved in a ninefold volume of 10 M LiBr, 100 mM Tris-HCl (pH 9.0), and the absorbance at 280 nm was measured. The protein concentration was determined based on a calibration curve obtained from fibroin protein.

### 2-4. Observation of Silk Nanoparticles with Scanning Electron Microscope (SEM) (1)

The silk nanoparticles obtained in (2-3) were diluted 10 times with RO water, and 1 µl of the diluted solution was dropped onto a silicon wafer to prepare a sample for observation. The observation was performed using a tabletop microscope (HITACHI TM2000Plus) under conditions at 5 kV and 2500x magnification.

### 2-5. Confirmation of Incorporation of Antigenic Protein into Silk Nanoparticles (1)

The "OVA-containing cocoon fiber nanoparticles" or the "OVA-containing degummed fiber nanoparticles" prepared in (2-3) were dissolved in 10 M LiBr, 100 mM Tris-HCl (pH 9.0), and the concentration of each of the silk aqueous solutions was adjusted to 2 mg/ml (in 2 M LiBr, 20 mM Tri-HCl (pH 9.0)). The sample was then diluted step-wise with SDS sample buffer, and 5, 2.5, 1, 0.5, or 0.25 µg of the sample was placed in each well. In addition, a portion of the supernatant of the nanoparticle suspension that had been stored at 4°C for about one week was collected and diluted 1/20 with SDS sample buffer, and 5 µl of this and the above sample were electrophoresed together. After SDS-PAGE, the resultant sample was transferred to a PVDF membrane, reacted with anti-OVA antibody (Abcam) (room temperature, 60 minutes), and then reacted with an alkaline phosphatase-labeled anti-rabbit antibody (Dako) (room temperature, 60 minutes). With addition of a BCIP-NBT solution (NACALAI TESQUE, INC.), the resultant sample was subjected to a coloring reaction.

### 2-6. Preparation of Silk Nanoparticles (2)

In addition, smaller-scaled silk nanoparticles were prepared by using various salting-out solvents adjusted to a concentration of 1.25 M instead of the 1.25 M K₂HPO₄ mentioned above. Specifically, in a 2 ml tube, 1.5 ml of each of the various salting-out solvents (trisodium citrate, sodium sulfate, magnesium sulfate, magnesium acetate, sodium acetate, ammonium sulfate, potassium acetate, sodium tartrate, and triammonium citrate) adjusted in advance to a concentration of 1.25 M was placed, 0.3 ml of the above undiluted solution for nanoparticles was superposed thereon, and the resultant was left to stand for 30 minutes. Then, the resultant was mixed by slow inversion, and left to stand at 4°C overnight to form nanoparticles. After being left to stand, the resultant was centrifuged (TOMY MX-307, 5,000 rpm, 15°C, 20 minutes) to remove the supernatant, 1.5 ml of RO water was added to the precipitate, mixed by inversion, centrifuged (TOMY MX-307, 15,000 rpm, 15°C, 20 minutes) to remove the supernatant, 250 µl of RO water was added to the precipitate, followed by ultrasonic disruption (BRANSON SONIFIRE) to obtain a silk nanoparticle solution.

### 2-7. Observation of Silk Nanoparticles with SEM (2)

On a silicon wafer, 1 µl of the silk nanoparticle solution obtained in (2-6) was dropped and dried to prepare a sample for observation. The observation was performed using a tabletop microscope (HITACHI TM4000Plus) under conditions at 5 kV and 2500x magnification.

### 2-8. Confirmation of Incorporation of Antigenic Protein into Silk Nanoparticles (2)

150 µl of the silk nanoparticle solution prepared in (2-6) was taken and centrifuged (15,000 rpm, 15°C, 10 minutes), 140 µl of the supernatant was discarded (10 µl was left), and 40 µl of 10 M LiBr, 100 mM Tris-HCl (pH 9.0) was added to completely dissolve the nanoparticles. The protein concentration was calculated by measuring A280, and the dissolving solution was adjusted to 1.5 mg/ml in 2 M LiBr, 20 mM Tris-HCl (pH 9.0).

An equal volume of 2X SDS sample buffer was added, the resultant sample was boiled, and 3 µg of the sample was placed in each well. After SDS-PAGE, the resultant sample was transferred to a PVDF membrane, reacted with anti-OVA antibody (Abcam) (room temperature, 60 minutes), and then reacted with horseradish peroxidase (HRP) labeled anti-rabbit antibody (Dako) (room temperature, 60 minutes). With addition of a chemiluminescence analysis reagent Chemi-Lumi One L (NACALAI TESQUE, INC.), the resultant sample was subjected to chemiluminescence, and then developed using Hyperfilm ECL (Cytiva).

### 3. Oral Administration Test of OVA-Containing Silk Nanoparticles to Mice

3-1. The four types of silk nanoparticles (approximately 35 mg/ml) prepared in (2-3) described above were orally administered using oral probes at 10 ml/kg (mouse body weight) per administration. An equal volume of normal saline was orally administered as a control. After three consecutive days of oral administrations, the silk nanoparticles were orally administered three times at weekly intervals from the first administration, and whole blood was collected under isoflurane inhalation anesthesia on the next day after the final administration. Three 6-week-old female ICR mice were used in each of the test and control groups.

### 3-2. ELISA Using Serum of Mice Subjected to Oral Administration with OVA-Containing Silk Nanoparticles

In a 96-well assay plate, 100 µl of the dissolving solution of the non-degummed cocoon fiber, the degummed fiber, or the sericin hope (0.25 mg/ml in each) or the OVA solution (0.625 mg/ml) was dispensed into each well, which cell was then coated with it at 4°C overnight. Each well was blocked with an assay diluent (Biolegend) (left to stand at room temperature). After washing with PBST (PBS containing 0.05% Tween 20), the resultant was reacted with the mouse serum diluted at 1/100 with the assay diluent (room temperature, 90 minutes). After washing, the resultant was reacted with HRP-labeled anti-mouse immunoglobulin antibody (Dako) or HRP-labeled anti-mouse IgA antibody (Abecam) (room temperature, 60 minutes). After washing, an ELISA POD substrate TMB solution (NACALAI TESQUE, INC.) was added. After color development, the reaction was stopped by adding 2 N H₂SO₄, and the absorbance (450 nm) was measured (Bio-Rad, iMark Microplate Reader).

The results obtained with the materials and the methods described above are shown below.

### (Results)

### (Comparative Example 1) Oral Administration Test Using Cocoons produced by genetically recombinant silkworms as Immunogen

Cocoons produced by recombinant silkworms expressing fibroin L chain-fused OVA protein (FibL-OVA) or cocoons produced by recombinant silkworms expressing free OVA in the sericin layer (Ser-OVA) were completely dissolved in 9 M lithium bromide. The cocoon fiber dissolving solution was subjected to SDS-PAGE and then Western blotting using anti-OVA antibody (Abcam) (primary antibody: anti-OVA antibody, and secondary antibody: alkaline phosphatase-labeled anti-rabbit polyclonal antibody). As a result, it was confirmed that both the FibL-OVA fusion protein and the free OVA in the sericin layer (Ser-OVA) were expressed in the cocoon fibers produced by the recombinant silkworms (Fig. 1).

Then, silk microparticles prepared by crushing the cocoons expressing FibL-OVA or Ser-OVA were orally administered to mice. As a result, it was confirmed that significant antibody production against OVA was not induced in the serum of these mice. On the other hand, strong antibody production against silk components, especially sericin was induced (Fig. 2).

The above results suggest that the oral administration of the crushed cocoons produced by genetically recombinant silkworms expressing the antigenic protein, prepared as described in JP 2005-97229 A, resulted in induction of predominant antibody production against the silk components, making it difficult to efficiently induce antibody production against the target antigen.

### (Example 1) Oral Administration Test to Mice Using OVA-Containing Silk Nanoparticles as Immunogen

A mixture solution in which OVA in a volume equal to 10% of the mass of the silk protein was added to the cocoon dissolving solution or the degummed fiber dissolving solution is mixed with 1.25 M K₂HPO₄ to prepare silk nanoparticles (Fig. 3A). The obtained silk nanoparticles were observed with a scanning electron microscope (SEM). As a result, the particles prepared from the degummed fiber had the largest diameters ranging from 0.5 to 4 µm, and overall the majority of the particles had the size of about 2 µm in diameter. On the other hand, in the nanoparticles prepared with addition of 10% OVA, the particle size distribution was almost the same, but many particles with slightly smaller diameters of 0.5 to 1.0 µm were observed. In the case where the cocoon dissolving solution or the cocoon dissolving solution containing 10% OVA was used as the raw material, the particles became even smaller, with most particles being 0.2 to 0.3 µm in diameter (Fig. 3B).

In addition, in order to confirm that OVA was incorporated into the prepared silk nanoparticles, the prepared silk nanoparticles were dissolved again in lithium bromide, and the resulting dissolving solution was subjected to SDS-PAGE and Western blotting using anti-OVA antibody, confirming that OVA was certainly incorporated into the silk nanoparticles (Fig. 4).

Furthermore, a suspension of the silk nanoparticles prepared by using the cocoon dissolving solution containing 10% OVA or the degummed fiber dissolving solution containing 10% OVA was stored at 4°C for approximately one week and then the presence of OVA in the supernatant of the suspension was confirmed. This revealed that OVA once incorporated into silk nanoparticles was gradually released into the solution in which the nanoparticles were suspended (Fig. 4).

Then, the silk nanoparticles prepared from the cocoon dissolving solution, the cocoon+OVA dissolving solution, the degummed fiber dissolving solution, or the degummed fiber+OVA dissolving solution were orally administered to mice, and whether antibody production against the silk components or OVA was induced was evaluated using the serum of the mice subjected to the oral administration.

As a result, the antibody (IgG and IgA) production specific to the sericin, which is a silk component, was observed in the serum of the mice subjected to the oral administration with the nanoparticles consisting only of the cocoons. In addition, slight reactions with the degummed fiber dissolving solution from which the sericin had been removed were observed, indicating that the antibody production against the fibroin was also induced (Figs. 5A and 5B).

On the other hand, in the mice subjected to the oral administration with the nanoparticles prepared from the cocoon+OVA dissolving solution, it was clarified that the antibody (IgG and IgA) production specific to the sericin was induced and the antibody (IgG and IgA) production specific to the OVA was further induced (Figs. 5A and 5B).

Furthermore, in the mice subjected to the oral administration with the nanoparticles consisting only of the degummed fiber or the nanoparticles prepared from the degummed fiber+OVA dissolving solution, the antibody production not only against the removed sericin but also against the fibroin was not induced (Figs. 5A and 5B).

Moreover, in the mice subjected to the oral administration with the nanoparticles prepared from the degummed fiber+OVA dissolving solution, the antibody (IgG and IgA) production specific to the OVA was remarkably induced (Fig. 5A and 5B).

### (Example 2) Formation of OVA-Containing Silk Nanoparticles Using Various Salting-Out Solvents

As described in (2-6) above, the mixture solution in which the OVA in the volume equal to 10% of the mass of the silk protein was added to the cocoon dissolving solution or the degummed fiber dissolving solution was mixed with each of the various salting-out solvents adjusted to 1.25 M to prepare the silk nanoparticles. As a result of observing the obtained silk nanoparticles with the SEM, it was confirmed that particles formed by using only the degummed fiber dissolving solution (DEGUMMED FIBER-OVA) had an average diameter of approximately 1 to 4 µm. On the other hand, in the case of the degummed fiber dissolving solution to which 10% OVA was added (DEGUMMED FIBER+OVA), it was confirmed that slightly smaller particles with diameters ranging from 0.8 to 3 µm were formed. In the case where only the cocoon dissolving solution (COCOON-OVA) or the cocoon dissolving solution to which 10% OVA was added (COCOON+OVA) was used as the raw material, it was confirmed that even smaller particles with an average diameter of 0.3 µm or less to approximately 0.5 µm were formed (Fig. 6).

In addition, in order to confirm that the OVA was incorporated into the silk nanoparticles thus prepared, the prepared silk nanoparticles were dissolved again in lithium bromide, and the resulting solution was subjected to SDS-PAGE and then Western blotting using anti-OVA antibody. As a result, it was confirmed that the OVA was certainly incorporated into the silk nanoparticles prepared by using the different salting-out solvents (Fig. 7).

The above results revealed that antibody production specific to an antigen can be induced by orally administering silk nanoparticles containing the antigen prepared from a mixture solution of the antigenic protein and a silk solution in which degummed fiber is dissolved (solution containing fibroin) or a silk solution in which non-degummed cocoon fiber is dissolved (solution containing sericin and fibroin).

### [Industrial Applicability]

As described above, according to the present invention, it is possible to induce antibody production specific to an antigen by oral administration of nanoparticles containing the antigen and fibroin. In particular, in the antibody production, the induction of antigen-specific production of both IgA, which exhibits activation of mucosal immunity, and IgG, which exhibits activation of systemic immunity, was observed. In other words, the nanoparticles deliver an antigen to the intestinal tracts (small intestine and large intestine) while avoiding decomposition of the antigen by gastric acid and digestive enzymes in the stomach, thereby making it possible to activate mucosal immunity and also induce systemic immunity. Therefore, the present invention is useful as a composition for an oral vaccine.

## Claims

1. A composition for an oral vaccine comprising nanoparticles containing an antigen and fibroin.

2. The composition for an oral vaccine according to claim 1, wherein the nanoparticles further contain sericin.

3. The composition for an oral vaccine according to claim 1, wherein the nanoparticles are precipitated by salting out from a mixture solution of a dissolving solution of degummed silk and the antigen.

4. The composition for an oral vaccine according to claim 2, wherein the nanoparticles are precipitated by salting out from a mixture solution of a dissolving solution of non-degummed silk fiber and the antigen.
